**Europäisches Patentamt**

**European Patent Office**

**Office européen des brevets**

(19)

(11) Veröffentlichungsnummer : **0 033 099**
**B1**

(12) **EUROPÄISCHE PATENTSCHRIFT**

(45) Veröffentlichungstag der Patentschrift :
**16.02.83**

(51) Int. Cl.³ : **C 07 C 91/44**

(21) Anmeldenummer : **81100291.4**

(22) Anmeldetag : **16.01.81**

(54) **Verfahren zur Herstellung von 2-Amino-4-nitrophenol.**

(30) Priorität : **23.01.80 DE 3002254**

(43) Veröffentlichungstag der Anmeldung :
**05.08.81 Patentblatt 81/31**

(45) Bekanntmachung des Hinweises auf die Patenterteilung : **16.02.83 Patentblatt 83/07**

(84) Benannte Vertragsstaaten :
**BE CH DE FR GB IT LI NL**

(56) Entgegenhaltungen :
**AT B 226 217**
**DE A 2 614 825**

(73) Patentinhaber : **CASSELLA Aktiengesellschaft**
**Hanauer Landstrasse 526**
**D-6000 Frankfurt am Main 61 (DE)**

(72) Erfinder : **Bauer, Wolfgang, Dr.**
**Masurenstrasse 6**
**D-6457 Maintal 3 (DE)**
Erfinder : **Kühlein, Klaus, Dr.**
**Fasanenstrasse 41**
**D-6233 Kelkheim/Taunus (DE)**

(74) Vertreter : **Urbach, Hans-Georg, Dr. et al**
**Hanauer Landstrasse 526**
**D-6000 Frankfurt am Main 61 (DE)**

Verfahren zur Herstellung von 2-Amino-4-nitrophenol

Die Erfindung betrifft ein Verfahren zur Herstellung von 2-Amino-4-nitrophenol durch Reduktion von 2,4-Dinitrophenol mit Hydrogensulfid in wäßrig alkalischer Lösung bei Temperaturen von 20 bis 100 °C.

Es ist bekannt, daß 2-Amino-4-nitrophenol durch partielle Reduktion von 2,4-Dinitrophenol hergestellt werden kann. Die elektrolytische Reduktion (Ber. dtsch. chem. Ges. *41*, 3 196 (1908)) liefert eine Ausbeute von 13 bis 53 % der Theorie ; die Reduktion mit Schwefeldioxid in Gegenwart von Eisenspänen (DE-PS 289 454) liefert eine Ausbeute von 60 % der Theorie ; die Reduktion mit Hydrazin in Gegenwart von Kupfer- oder Eisen-Pulver (J. Pharm. Soc. Japan *76*, 801 (1956) zitiert nach Chemical Abstracts *51*, 1 087 (1957)) in ethanolischer Lösung liefert eine Ausbeute von 75 % der Theorie. Die vorgenannten Verfahren liefern das gewünschte Produkt nur in ungenügenden Ausbeuten oder besitzen andere Nachteile, wie z. B. den Einsatz hoher Metallmengen, Verwendung organischer Lösungsmittel oder gefährlicher Reaktionspartner, mangelnde Arbeitssicherheit oder hohe Umweltbelastung. Andere bekannte Verfahren verwenden zur partiellen Reduktion des 2,4-Dinitrophenols sulfidische Reduktionsmittel, z. B. eine Mischung von Ammonium-sulfid und Schwefelwasserstoff (K. Auwers u. H. Röhrig, Ber. dtsch. chem. Ges. *30*, 988 (1897), Ausbeute 32 % der Theorie) ; Dinatriumtrisulfid (G. I. Gershzon, J. applied Chem. (USSR) *9*, 879 (1936), vgl. Chem. Abstracts *30*, 7 554 (1936), Ausbeute 70 % der Theorie) ; Dinatriumdisulfid (P. E. Verkade et al Rec. trav. Chem. *65*, 346 (1946) und GB-PS 594 816, Ausbeute 59 % der Theorie) ; Natriumsulfid (E. Marval u. J. Urbik, CS-PS 90 290, vgl. Chem. Abstracts *54*, P 24 552, Ausbeute ca. 60 % der Theorie, und Organic Synthesis, Col. Vol. III, Ausbeute 64 bis 67 % der Theorie). Sofern die vorgenannten, mit sulfidischen Reduktionsmitteln arbeitenden Verfahren überhaupt vernünftige Ausbeuten der gewünschten Verbindung liefern, entsteht dabei gleichzeitig unerwünschterweise das isomere 4-Amino-2-nitrophenol, das durch besondere Reinigungsoperationen abgetrennt werden muß. Zum Teil entstehen als unerwünschte Nebenprodukte auch Schwefelfarbstoffe, die auf apparativ aufwendige und teuere Weise abgetrennt werden müssen. Unter Verwendung von Alkalimetallsulfiden oder Polysulfiden soll eine selektive partielle Reduktion der in 2-Stellung stehenden Nitrogruppe des 2,4-Dinitrophenols möglich sein (US-PS 2 464 194), wenn das entsprechende Phenolat in hoher Verdünnung bei 20 bis 25 °C 12 Stunden und anschließend bei etwa 80 °C reduziert wird. Die geforderten hohen Verdünnungen und die langen Reaktionszeiten machen das Verfahren unwirtschaftlich.

Bei einem neueren Verfahren (US-PS 4 115 652 identisch mit DE-A1-2614825) wird 2,4-Dinitrophenol in einer wäßrigen ammoniakalischen Lösung, die pro Mol 2,4-Dinitrophenol, 1 bis 8 Mol Ammoniak und 2,5 bis 6 Mol Natriumhydrogensulfid enthält, bei Temperaturen von Raumtemperatur bis 100 °C reduziert. Die Ausbeuten liegen bei 74 bis 76 % der Theorie. Bei Nacharbeitungen dieses Verfahrens wurden in dem erhaltenen Rohprodukt 5 bis 10 % des unerwünschten Isomeren festgestellt, so daß auch bei diesem Verfahren eine Reinigung über das 2-Amino-4-nitrophenolat erfolgen muß.

Es wurde nun überraschenderweise gefunden, daß eine praktisch selektive, partielle Reduktion der in 2-Stellung stehenden Nitrogruppe des 2,4-Dinitrophenols mit Hilfe von Hydrogensulfid in wäßrig alkalischer Lösung bei Temperaturen von 20 bis 100 °C dann möglich ist, wenn während der Reduktion ein pH-Wert von 9,5 nicht überschritten wird.

Die bei dem erfindungsgemäßen Verfahren benutzten Hydrogensulfide können solche der Alkalimetalle (Lithium, Natrium, Kalium, Rubidium, Caesium), der Erdalkalimetalle (z. B. Calcium, Strontium, Barium) oder des Ammoniums sein. Vorzugsweise wird Natrium-, Kalium- oder Ammoniumhydrogensulfid verwendet. Auch Mischungen von zwei oder mehreren Hydrogensulfiden können eingesetzt werden. Normalerweise werden pro Mol 2,4-Dinitrophenol 3 bis 4 Mole Hydrogensulfid benötigt.

Die Reduktion wird in wäßrig alkalischer Lösung so durchgeführt, daß erfindungsgemäß während der gesamten Reduktion ein pH-Wert von 9,5, vorzugsweise 9,0, ganz besonders bevorzugt von 8,5, nicht überschritten wird. Der pH-Wert während der Reduktion liegt im alkalischen Bereich zwischen 7 und 9,5, vorzugsweise zwischen 7,1 und 9,0, ganz besonders bevorzugt zwischen 8,0 und 8,5. Freies 2,4-Dinitrophenol wird in Wasser unter Zusatz von soviel Lauge, z. B. Natron- oder Kalilauge, gelöst, daß sich ein pH-Wert von 7 bis 9,5 einstellt. Vorteilhafterweise kann das benötigte Ausgangsprodukt auch im Reaktionsansatz erzeugt werden, z. B. durch wäßrig alkalische Hydrolyse von 2,4-Dinitrochlorbenzol. Nach beendeter Hydrolyse wird der pH-Wert der Lösung gemessen und, falls erforderlich, auf 7 bis 9,5 eingestellt. Bei einer Reaktionstemperatur von 20 bis 100 °C, vorzugsweise 50 bis 80 °C, wird das Hydrogensulfid unter Kontrolle des pH-Werts des Reaktionsansatzes und zweckmäßigerweise unter Rühren zudosiert. Während der gesamten Dauer der Reduktion wird durch Zugabe geeigneter, Protonen liefernder Substanzen dafür gesorgt, daß ein pH-Wert von 9,5 vorzugsweise 9,0, ganz besonders bevorzugt von 8,5, nicht überschritten wird. Dadurch wird erreicht, daß während der gesamten Dauer der Reduktion ein alkalischer pH-Wert bis maximal 9,5, vorzugsweise 7,1 bis 9,0, ganz besonders bevorzugt von 8,0 bis 8,5, eingehalten wird. Geeignete, Protonen liefernde Substanzen sind z. B. anorganische oder organische Säuren, wie z. B. Schwefel-, Salz-, Phosphor-, Kohlen-, Essig-, Propion-, Benzoe-, Benzolsulfon-, p-Toluolsulfon-, Amidosulfon-, Carbamidsäure, Schwefelwasserstoff ; saure Salze, wie z. B. Natrium- oder Kaliumhydrogensulfat, Ammoniumhydrogencarbonat ; primäre und sekundäre Natrium-, Kalium- und Ammoniumphosphate oder Ammoniumsalze, wie z. B. Ammonchlorid, Ammonsulfat, Ammoncarbonat. Bei puffernd wirkenden, Protonen liefernden Substanzen, wie z. B. Ammoniumsalzen, ist es auch möglich, die zur Einstellung des pH-Wertes auf 7 bis 9,5 benötigte Menge in einem Vorversuch festzustellen und diese Menge dann insgesamt vor Beginn der Reduktion dem Reaktionsansatz zuzufügen. Pro Mol 2,4-Dinitrophenol werden bei Verwendung puffernd wirkender, Protonen liefernder Substanzen etwa 3 bis 5 Mole, in vielen Fällen etwa 3 bis 4 Mole, benötigt.

Die Verwendung von Schwefelwasserstoff als Protonen liefernde Substanz bietet darüberhinaus die Möglichkeit, das als Reduktionsmittel benötigte Hydrogensulfid im Reaktionsansatz zu erzeugen. Bei Verwendung von Schwefelwasserstoff zur Erzeugung von Hydrogensulfid im Reaktionsansatz ist es zweckmäßig, gleichzeitig alkalisch reagierende Verbindungen, wie Alkalihydroxid, Alkalicarbonat, tertiäres oder sekundäres Alkaliphosphat, zuzusetzen, um die bei dem erfindungsgemäßen Verfahren einzustellenden pH-Werte zu erhalten. Als Alkalihydroxid, Alkalicarbonat oder Alkaliphosphat werden dabei normalerweise die Natriumverbindungen benutzt.

Das erfindungsgemäße Verfahren wird vorzugsweise in Wasser als Lösungsmittel durchgeführt. Dem wäßrigen Reaktionsmedium können jedoch auch mit Wasser mischbare Lösungsmittel, beispielsweise aus der Reihe der ein- oder mehrwertigen Alkohole, wie Methanol, Ethanol, Isopropanol, Glykol, Glykoläther, Diethylenglykol, Triethylenglykol, oder mit Wasser nicht mischbare Lösungsmittel, beispielsweise Methylenchlorid, n-Hexan, Cyclohexan, Toluol, Monochlorbenzol, zugefügt werden. Ferner können dem Reaktionsmedium an sich bekannte Tenside, beispielsweise Aniontenside, Kationtenside oder nichtionogene Tenside (vgl. beispielsweise Ullmann's Enzyklopädie der Technischen Chemie, Band 16, Seiten 724 bis 748 (1965)) zugesetzt werden.

Nach dem erfindungsgemäßen Verfahren kann die selektive partielle Reduktion der in 2-Stellung stehenden Nitrogruppe des 2,4-Dinitrophenols innerhalb kurzer Reaktionszeiten bis herab zu etwa 30 Minuten oder noch kürzer durchgeführt werden. Bei dem bevorzugten Temperaturbereich von 50 bis 80 °C ergeben sich in der Regel Reaktionszeiten von 45 Minuten bis 2 Stunden. Nach beendeter partieller Reduktion liegt das Produkt als 2-Amino-4-nitrophenolat in dem Reaktionsansatz vor und wird normalerweise von dem bei der Reduktion gebildeten Schwefel abgetrennt. Für die Abtrennung des Schwefels und Isolierung des 2-Amino-4-nitrophenol können beispielsweise folgende Methoden angewandt werden.

1. Nach beendeter partieller Reduktion wird das 2-Amino-4-nitrophenolat durch Abkühlen und gegebenenfalls durch Aussalzen mittels eines Elektrolyten, wie Natriumchlorid, in Form eines schwefelhaltigen Produktes isoliert und anschließend wieder in einer verdünnten starken Säure, z. B. verdünnter Salzsäure, bei pH-Werten von 0 bis 2 gelöst. Dann wird vom Schwefel abfiltriert und im Filtrat das 2-Amino-4-nitrophenol durch Einstellung eines pH-Werts von 2 bis 6, vorzugsweise 4,5 bis 5,5, zur Abscheidung gebracht. Das abgeschiedene Produkt wird dann isoliert.

2. Nach beendeter partieller Reduktion wird das schwefelhaltige Reaktionsgemisch mit geeigneten Säuren, z. B. Salzsäure, auf einen pH-Wert von 2 bis 6, vorzugsweise 4,5 bis 5,5, eingestellt, abgekühlt und das abgeschiedene 2-Amino-4-nitrophenol zusammen mit dem Schwefel isoliert. Die Abtrennung des Schwefels erfolgt dann beispielsweise, wie bei Methode 1 beschrieben, durch Überführung des erhaltenen 2-Amino-4-nitrophenols mit wäßriger Salzsäure in eine Lösung leicht wasserlöslichen 2-Amino-4-nitrophenol-hydrochlorids und anschließende Abfiltration des Schwefels. Aus dem Filtrat wird das 2-Amino-4-nitrophenol durch Einstellung eines pH-Werts von 2 bis 6, vorzugsweise 4,5 bis 5,5, zur Abscheidung gebracht und kann dann isoliert werden.

3. Nach beendeter partieller Reduktion wird das schwefelhaltige Reduktionsgemisch zur Entfernung des Schwefels a) im alkalischen Bereich filtriert oder b) durch Zusatz einer starken Säure, vorzugsweise verdünnter Salzsäure, auf pH-Werte von 0 bis 2 gestellt und dann filtriert. Der pH-Wert des Filtrats wird im Fall a) durch Zusatz von Säuren, wie z. B. Salzsäure, und im Fall b) durch Zusatz alkalisch reagierender Stoffe, wie z. B. Natronlauge oder Natriumcarbonat, auf pH-Werte von 2 bis 6, vorzugsweise 4,5 bis 5,5, eingestellt und das 2-Amino-4-nitrophenol dadurch zur Abscheidung gebracht, das dann isoliert werden kann.

Bei den verschiedenen Aufarbeitungsmöglichkeiten ist es zweckmäßig, in den alkalischen, neutralen oder sauren Lösungen vorhandene geringe Verunreinigungen, beispielsweise geringe Schwefelmengen, durch chemische Reaktionen zu entfernen. Beispielsweise kann der Schwefel durch Zusatz eines Sulfits, normalerweise eines Alkalisulfits, vorzugsweise von Natriumsulfit, eines Pyrosulfits, normalerweise Alkalipyrosulfits, insbesondere von Natriumpyrosulfit, oder eines Hydrogensulfits, normalerweise eines Alkalihydrogensulfits, vorzugsweise von Natriumhydrogensulfit in lösliches Thiosulfat überführt werden. Anstelle des Zusatzes eines Sulfites kann auch Schwefeldioxid in die alkalische Lösung eingeleitet werden. Neben Sulfit, bzw. Schwefeldioxid, Pyrosulfit und Hydrogensulfit kann auch ein Hydrosulfit (= Dithionit, $S_2O_4^{2-}$), normalerweise ein Alkalidithionit, vorzugsweise Natriumdithionit, verwendet werden.

Die direkte Abscheidung und nachfolgende Isolierung des 2-Amino-4-nitrophenols nach den vorstehend beschriebenen Methoden 2 und 3 durch Einstellung eines pH-Werts von 2 bis 6, vorzugsweise 4,5 bis 5,5, ohne den Umweg über das 2-Amino-4-nitro-phenolat, ist möglich, weil bei dem erfindungsgemäßen Verfahren praktisch keine Nebenprodukte, wie isomeres 4-Amino-2-nitrophenol oder Schwefelfarbstoffe, entstehen. (Die genannte Abscheidungsmethode kann auch in anderen Fällen, in denen praktisch reines 2-Amino-4-nitrophenol vorliegt, von Vorteil sein). Das erfindungsgemäße Verfahren liefert 2-Amino-4-nitrophenol in überraschend hohen Ausbeuten von 85 bis 96 % der Theorie und in hervorragender Reinheit. Durch die Unterdrückung der Bildung von Schwefelfarbstoffen und des unerwünschten, Abwasser belastenden 4-Amino-2-nitrophenols fallen bei dem erfindungsgemäßen Verfahren auch ökologisch günstigere Abwasser an.

Die folgenden Beispiele dienen zur weiteren Erläuterung des erfindungsgemäßen Verfahrens. Teile bedeuten Gewichtsteile, Prozente Gewichtsprozente ; die Temperaturen sind in Celsiusgraden angegeben. Die angegebenen Schmelzpunkte sind nicht korrigiert.

Beispiel 1

Eine Suspension von 202,6 Teilen 2,4-Dinitrochlorbenzol in 300 Teilen Wasser und 7 Teilen 32,6 %iger Natroniauge wird auf ca. 90 °C erhitzt und bei 90 bis 95 °C unter Rühren im Verlauf von ca. 3 Stunden mit 256 Teilen 32,6 %iger Natronlauge versetzt. Man rührt ca. 2 Stunden bei 90 bis 95 °C nach und gibt zu der erhaltenen 2,4-Dinitrophenolat-Suspension 400 Teile Wasser und 184 Teile Ammoniumchlorid, wodurch sich ein pH-Wert von 7,0 einstellt. Dann werden bei 70 bis 75 °C unter ständigem Rühren und ständiger Kontrolle des pH-Werts im Verlauf von ca. 45 Minuten 580 Teile einer 31,9 %igen Sulfhydratlauge zudosiert, wobei der pH-Wert des Reduktionsansatzes auf pH 8,6 steigt. Man rührt ca. 45 Minuten bei 70 bis 75 °C nach. Anschließend streut man 40 Teile Natriumchlorid ein, kühlt das Reaktionsgemisch auf ca. 5 °C ab und filtriert. Man erhält 410 Teile eines 39,6 %igen wäßrigen Preßkuchens von Natrium-2-amino-4-nitrophenolat, entsprechend 92 % der theoretischen Ausbeute. 410 Teile dieses Preßkuchens werden in 1 000 Teile Wasser eingetragen und mit 225 Teilen 30 %iger wäßriger Salzsäure bei pH-1 gelöst. Anschließend filtriert man vom Schwefel ab und versetzt die wäßrige Lösung des 2-Amino-4-nitrophenol-hydrochlorids mit 116 Teilen 32,6 %iger Natronlauge, um einen pH von 5 einzustellen. Man rührt die gelbe Suspension des 2-Amino-4-nitrophenols ca. 1 Stunde bei 5 bis 10 °C nach und isoliert das abgeschiedene Produkt durch Filtration. Man erhält 324 Teilen eines 41,9 %igen wäßrigen Preßkuchens von 2-Amino-4-nitrophenol, entsprechend 135,8 Teilen 2-Amino-4-nitrophenol 100 %ig (88 % der Theorie, bezogen auf 2,4-Dinitrochlorbenzol). Eine getrocknete Probe weist einen Schwefelgehalt von < 0,1 % und einen Schmelzpunkt von 144 °C auf.

Beispiel 2

202,6 Teile 2,4-Dinitrochlorbenzol werden nach den Angaben des Beispiels 1 in Natrium-2,4-dinitrophenolat überführt und nach Zugabe von 400 Teilen Wasser und 184 Teilen Ammoniumchlorid ein pH-Wert von 7,0 eingestellt. Unter Rühren und unter pH-Kontrolle wird bei pH-Werten von maximal 9 mit 580 Teilen 31,9 %iger Sulfhydratlauge zu Natrium-2-amino-4-nitrophenolat reduziert. Nach der Sulfhydratzugabe rührt man ca. 45 Minuten bei 70 bis 75 °C nach und filtriert vom ausgefallenen Schwefel ab. Das Filtrat wird mit 100 g Natriumsulfit versetzt, auf ca. 15 °C gekühlt und mit 136 Teilen 30 %iger Salzsäure auf pH = 5,6 bis 5,8 gestellt. Man rührt noch ca. 1 Stunde bei 5 bis 10 °C nach filtriert und wäscht den Filterkuchen mit ca. 200 Teilen 26 %iger wäßriger Natriumchloridlösung nach. Man erhält 277,6 Teile eines 53,3 %igen wäßrigen Preßkuchens von 2-Amino-4-nitrophenol. entsprechend 147,9 Teilen 2-Amino-4-nitrophenol 100 %ig (96 % der Theorie, bezogen auf 2,4-Dinitrochlorbenzol). Das Produkt hat einen Schwefelgehalt von < 0,1 % und einen Schmelzpunkt von 144 °C.

Beispiel 3

Die Herstellung von Natrium-2,4-dinitrophenolat aus 202,6 Teilen 2,4-Dinitrochlorbenzol erfolgt nach den Angaben des Beispiels 1. Anschließend wird die Sulfhydratreduktion der mit 400 Teilen Wasser und 184 Teilen Ammoniumchlorid versetzten 2,4-Dinitrophenolat-Suspension entsprechend den Angaben des Beispiels 1 durchgeführt. Das schwefelhaltige Reaktionsgemisch wird nach der Reduktion mit 216 Teilen 30 %iger Salzsäure auf pH = 7 gestellt und zur Vervollständigung der Schwefelfällung ca. 1 Stunde bei 40 bis 45 °C nachgerührt. Anschließend wird mit 135 Teilen 32,6 %iger wäßriger Natronlauge auf pH = 9 gestellt und vom Schwefel abfiltriert. Das rote Filtrat des Natrium-2-amino-4-nitrophenolats wird zunächst mit 43,6 Teilen Natriumsulfit versetzt und dann mit 158 Teilen 30 %iger Salzsäure auf pH = 5,6 gestellt. Man rührt die wäßrige 2-Amino-4-nitrophenol-Suspension ca. 1 Stunde bei 5 bis 10 °C nach und isoliert das Produkt durch Filtration.

Man erhält 228 Teile eines 62,3 %igen wäßrigen Preßkuchens von 2-Amino-4-nitrophenol, entsprechend 142 Teilen 2-Amino-4-nitrophenol 100 %ig (92 % der Theorie ; bezogen auf 2,4-Dinitrochlorbenzol) ; Schwefelgehalt : < 0,1 % Schmelzpunkt : 143 bis 144 °C.

Beispiel 4

Man verfährt nach den Angaben des Beispiels 3, setzt jedoch kein Ammoniumchlorid zu, sondern hält pH-Wert während der Reduktion durch laufende Zugabe von insgesamt 330 Teilen 50 %ige Eissigsäure im Bereich von 8 bis 9,2.

Man erhält 230,5 Teile einer 59,5 %igen wäßrigen Paste von 2-Amino-4-nitrophenol, entsprechend 137,1 Teilen 2-Amino-4-nitrophenol 100 %ig (89 % der Theorie, bezogen auf 2,4-Dinitrochlorbenzol).

Beispiel 5

Verfährt man nach den Angaben des Beispiels 3, setzt jedoch kein Ammoniumchlorid zu, sondern hält den pH-Wert während der Reduktion durch laufende portionenweise Zugabe von insgesamt 200 Teilen Natrium-dihydrogenphosphat im Bereich von 8 bis 9,0. Man erhält 720 Teile eines 19,3 %igen wäßrigen Preßkuchens von 2-Amino-4-nitrophenol, entsprechend 139 Teilen 2-Amino-4-nitrophenol 100 %ig. Ausbeute 90 % der Theorie, bezogen auf eingesetztes 2,4-Dinitrochlorbenzol.

Beispiel 6

Eine Suspension von 184,1 Teilen 2,4-Dinitrophenol in 250 Teilen Wasser wird auf 70 °C erhitzt und mit 127 Teilen 30,2 %iger wäßriger Natronlauge versetzt. Anschließend stellt man durch Zugabe von 35,6 Teilen Ammoniumchlorid einen pH-Wert von 7,0 ein. Im Verlauf von ca. 45 Minuten gibt man anschließend unter Rühren und Kontrolle des pH-Werts 580 Teile einer 31,9 %igen wäßrigen Sulfhydratlauge zu und trägt während des Sulfhydratlauge-Zulaufs portionsweise insgesamt weitere 197 Teile Ammoniumchlorid ein. Dadurch wird während der gesamten Zeit der Reduktion ein pH-Wert von 8,6 nicht überschritten. Man läßt noch ca. 45 Minuten nachrühren, kühlt das Gemisch auf 20 bis 25 °C ab und streut 35 Teile Natriumsulfit ein. Danach stellt man den pH-Wert des Reduktionsgemischs durch langsame Zugabe von 244 Teilen 30 %iger Salzsäure auf pH 5 und rührt ca. 1 1/2 Stunden bei 5 bis 10 °C nach. Anschließend wird filtriert und der schwefelhaltige Preßkuchen von 2-Amino-4-nitrophenol mit 100 Teilen 26 %iger Natriumchlorid-Lösung gewaschen.

Der Preßkuchen wird dann in 400 Teile Wasser eingetragen und mit 53 Teilen Natriumcarbonat versetzt. Anschließend heizt man auf 50 bis 55 °C und entfernt den Schwefel durch Filtration. Das Filtrat wird mit ca. 98 Teilen 30 %iger Salzsäure auf pH = 5 gestellt und die 2-Amino-4-nitrophenol-Suspension ca. 2 Stunden bei ca. 5 °C nachgerührt. Nach der Filtration erhält man 346 Teile eines 41 %igen wäßrigen Filterkuchens von 2-Amino-4-nitrophenol, entsprechend 141,9 Teilen 2-Amino-4-nitrophenol 100 %ig. Ausbeute 92 % der Theorie, bezogen auf 2,4-Dinitrophenol.

Beispiel 7

Die Herstellung von Natrium-2,4-dinitrophenolat aus 202,6 Teilen 2,4-Dinitrochlorbenzol erfolgt nach den Angaben des Beispiels 1. Die Suspension des 2,4-Dinitrophenolats wird bei 70 °C mit 92 Teilen Ammoniumchlorid versetzt, wobei sich ein pH-Wert von 7,1 einstellt. Anschließend werden unter Rühren und Kontrolle des pH-Wertes in ca. 20 Minuten 290,4 Teile 31,9 %ige wäßrige Sulfhydratlauge zugegeben. Durch erneute Zugabe von 92 Teilen Ammoniumchlorid senkt man den pH-Wert von 8,4 auf 8,0 und setzt dann bei 70 bis 75 °C in ca. 20 Minuten nochmals 290,4 Teile 31,9 %ige Sulfhydratlauge zu. Hierbei steigt der pH-Wert auf 8,4. Anschließend wird ca. 45 Minuten bei ca. 70 °C nachgerührt. Man kühlt die Reaktionsmischung auf 15 bis 20 °C, gibt 35 Teile Natriumsulfit zu und dann ca. 230 Teile einer 30 %igen wäßrigen Salzsäure, um auf pH = 5 zu stellen. Die schwefelhaltige 2-Amino-4-nitrophenol-Suspension wird ca. 1 1/2 Stunden bei 5 bis 10 °C nachgerührt und dann filtriert. Man erhält 438,4 Teile einer 34 %igen wäßrigen Paste von 2-Amino-4-nitrophenol mit einem Schwefelgehalt von ca 19 %. Dieser schwefelhaltige Filterkuchen wird in einer Lösung von 53 Teilen Natriumcarbonat in 400 Teile Wasser eingetragen. Man erwärmt auf ca. 55 bis 60 °C und entfernt den Schwefel aus der Lösung des Natrium-2-amino-4-nitrophenolats durch Filtration. Anschließend kühlt man das Filtrat auf ca. 20 °C und stellt mit ca. 107 Teilen 30 %iger wäßriger Salzsäure auf pH = 5. Nach Kühlen auf ca. 5 °C wird filtriert. Man erhält 344 Teile eines 42,1 %igen wäßrigen Preßkuchens von 2-Amino-4-nitrophenol, entsprechend 144,8 Teilen 2-Amino-4-nitrophenol 100 %ig. Ausbeute 94 % der Theorie, bezogen auf 2,4-Dinitrochlorbenzol.

Beispiel 8

Die Herstellung von Natrium-2,4-dinitrophenolat aus 202,6 Teilen 2,4-Dinitrochlorbenzol wird nach den Angaben des Beispiels 1 durchgeführt. Die Suspension des 2,4-Dinitrophenolats wird mit 30 %iger wäßriger Salzsäure auf pH 7,5 gestellt. Anschließend werden bei 70 bis 75 °C in ca. 1 Stunde 580 Teile eines 31,4 %igen Sulfhydratlauge und gleichzeitig 259 Teile 30 %ige, wäßrige Salzsäure zudosiert, wobei ein pH-Wert von 8,5 nicht überschritten wird. Nach beendeter Reduktion wird vom Schwefel abfiltriert. Die Natrium-2-amino-4-nitrophenolat-Lösung wird mit 70 Teilen Natriumsulfit (alternativ : 52,3 Teile Natriumpyrosulfit oder 57,2 Teile Natriumhydrogensulfit) versetzt und dann mit 30 %iger Salzsäure auf pH 3,8 gestellt. Anschließend kühlt man die 2-Amino-4-nitrophenol-Suspension auf 0 bis 5 °C und isoliert das Produkt durch Filtration. Ausbeute 202 g 2-Amino-4-nitrophenol 71,5 %ig, entsprechend 144,4 g 2-Amino-4-nitrophenol 100 %-ig (93 % der Theorie, bezogen auf 2,4-Dinitrochlorbenzol).

Eine getrocknete Probe weist einen Schmelzpunkt von 144 °C auf.

Leitet man anstelle des Natriumsulfit-, Natriumpyrosulfit-bzw. Natriumhydrogensulfit-Zusatzes nach der Schwefelfiltration Schwefeldioxid in die schwach alkalische 2-Amino-4-nitro-phenolat-Lösung bis zu einem pH-Wert von 4,5 ein, so erhält man 2-Amino-4-nitrophenol ebenfalls in einer Ausbeute von 93 % der Theorie.

Beispiel 9

Die Herstellung von Natrium-2,4-dinitrophenolat und die Sulfhydrat-Reduktion zu Natrium-2-amino-4-nitrophenolat wird nach den Angaben des Beispiels 8 durchgeführt. Nach beendeter Reduktion wird die schwefelhaltige 2-Amino-4-nitrophenolat-Lösung zu 310 Teilen 30 %iger wäßriger Salzsäure gegeben. Die Lösung des 2-Ami-no-4-nitro-phenol-hydrochlorids wird vom Schwefel abfiltriert und mit 8 Teilen Hydrosulfit konz. (Natriumdithionit) versetzt. Anschließend wird mit 30 %iger Natronlauge ein pH-Wert von 3,9 eingestellt und die 2-Amino-4-nitrophenol-Suspension auf 0-5 °C gekühlt. Nach Filtration erhält man 190 g 2-Amino-4-nitrophenol 74,8 %ig, entsprechend 142,1 g 2-Amino-4-nitrophenol 100 %ig (92 % der Theorie, bezogen auf 2,4-Di-nitrochlorbenzol).

Beispiel 10

Die Herstellung von Natrium-2,4-dinitrophenolat aus 202,6 Teilen 2,4-Dinitrochlorbenzol erfolgt nach den Angaben des Beispiels 1. Anschließend setzt man 150 Teile Wasser zu, kühlt auf 65 bis 70 °C und stellt mit 8 Teilen 30 %iger Salzsäure auf pH 8.

Anschließend werden im Verlauf von ca. 3 Stunden bei Normaldruck (alternativ im Verlauf von 1 Stunde in einem Druckgefäß bei max. 5 bar) bei 65 bis 70 °C 102 g Schwefelwasserstoff und gleichzeitig 30 Teile 30 %ige Natronlauge zudosiert, wobei sich ein pH-Wert von 8 bis 8,5 einstellt.

Nach der Schwefelwasserstoffzugabe rührt man noch ca. 15 Minuten bei 65 bis 70 °C nach. Anschließend wird die schwefelhaltige 2-Amino-4-nitrophenolat-Lösung zu 310 Teilen 30 %iger Salzsäure gegeben.

Die Lösung des 2-Amino-4-nitrophenol-hydrochlorids wird bei 60 bis 70 °C filtriert und mit einer Lösung von 25 g Natriumsulfit in 240 Teilen 30 %iger Natronlauge vermischt. Anschließend stellt man einen pH-Wert von 3,6 bis 4 ein, kühlt die 2-Amino-4-nitrophenol-Suspension auf 0 bis 5 °C, filtriert und wäscht mit wenig Eiswasser nach.

Ausbeute : 176,5 g 2-Amino-4-nitrophenol 74,8 %ig, entsprechend 132 g 2-Amino-4-nitrophenol 100 %ig (86 % der Theorie, bezogen auf 2,4-Dinitrochlorbenzol).

Die in den vorstehenden Beispielen verwendete Sulfhydratlauge ist eine wäßrige Lösung von Natriumhydrogensulfid.

Das 2-Amino-4-nitrophenol wird beispielsweise als Zwischenprodukt zur Herstellung von Metall-komplex-Farbstoffen verwendet.

## Ansprüche

1. Verfahren zur Herstellung von 2-Amino-4-nitrophenol durch Reduktion von 2,4-Dinitrophenol mit Hydrogensulfid in wäßrig alkalischer Lösung bei Temperaturen von 20 bis 100 °C, dadurch gekennzeichnet, daß während der Reduktion ein pH-Wert von 9,5 nicht überschritten wird.

2. Verfahren nach Anspruch 1, dadurch gekennzeichnet, daß während der Reduktion ein pH-Wert von 9,0, vorzugsweise 8,5, nicht überschritten wird.

3. Verfahren nach den Ansprüchen 1 oder 2, dadurch gekennzeichnet, daß während der Reduktion ein alkalischer pH-Wert von 7 bis 9,5, vorzugsweise 7,1 bis 9,0, ganz besonders bevorzugt von 8,0 bis 8,5, eingehalten wird.

4. Verfahren nach den Ansprüchen 1 bis 3, dadurch gekennzeichnet, daß die Reduktion bei einer Temperatur von 50 bis 80 °C durchgeführt wird.

5. Verfahren nach den Ansprüchen 1 bis 4, dadurch gekennzeichnet, daß zur Reduktion von 1 Mol 2,4-Dinitrophenol 3 bis 4 Mole eines Alkali- oder Erdalkalihydrogensulfids oder Ammoniumhydrogensulfids verwendet wird.

6. Verfahren nach den Ansprüchen 1 bis 5, dadurch gekennzeichnet, daß die Überschreitung des pH-Wertes von 9,5, vorzugsweise von 9,0, ganz besonders bevorzugt 8,5, durch portionsweise oder kontinuierliche Zugabe Protonen liefernder Substanzen verhindert wird.

7. Verfahren nach den Ansprüchen 1 bis 5, dadurch gekennzeichnet, daß die Überschreitung des pH-Wertes von 9,5, vorzugsweise von 9,0, ganz besonders bevorzugt 8,5, durch Zugabe einer puffernd wirkenden Substanz vor Beginn der Reduktion verhindert wird.

8. Verfahren nach den Ansprüchen 1 bis 7, dadurch gekennzeichnet, daß nach beendeter Reduktion der in der Lösung vorhandene Schwefel durch Filtration entfernt und in dem Filtrat das 2-Amino-4-nitrophenol durch Einstellung eines pH-Werts von 2 bis 6, vorzugsweise von 4,5 bis 5,5, zur Abscheidung gebracht wird.

9. Verfahren nach den Ansprüchen 1 bis 7, dadurch gekennzeichnet, daß nach beendeter Reduktion der in der Lösung vorhandene Schwefel abfiltriert und nach der Filtration noch vorhandene Verunreinigungen durch chemische Reaktion, vorzugsweise mit einem Alkalisulfit, Schwefeldioxid, Alkalipyrosulfit, Alkalihydrogensulfit oder Alkalidithionit, in Lösung gebracht und das 2-Amino-4-nitrophenol durch Einstellung eines pH-Werts von 2 bis 6, vorzugsweise von 4,5 bis 5,5 zur Abscheidung gebracht wird.

10. Verfahren nach den Ansprüchen 1 bis 9, dadurch gekennzeichnet, daß als Reduktionsmittel Natriumhydrogensulfid verwendet wird.

## Claims

1. Process for the preparation of 2-amino-4-nitrophenol by reduction of 2,4-dinitrophenol with hydrosulphide in aqueous alkaline solution at temperatures from 20 to 100 °C, characterised in that the pH value does not exceed 9.5 during the reduction.

2. Process according to Claim 1, characterised in that the pH value does not exceed 9.0, preferably 8.5, during the reduction.

3. Process according to Claim 1 or 2, characterised in that an alkali pH value of 7 to 9.5, preferably 7.1 to 9.0 and very particularly preferably 8.0 to 8.5, is maintained during the reduction.

4. Process according to Claims 1 to 3, characterised in that the reduction is carried out a temperature of 50 to 80 °C.

5. Process according to Claims 1 to 4, characterised in that 3 to 4 mols of an alkali metal hydrosulphide or alkaline earth metal hydrosulphide or of ammonium hydrosulphide are used for the reduction of 1 mol of 2,4-dinitrophenol.

6. Process according to Claims 1 to 5, characterised in that exceeding the pH value of 9.5, preferably of 9.0 and very particularly preferably of 8.5 is prevented by portionwise or continuous addition of substances which supply protons.

7. Process according to Claims 1 to 5, characterised in that exceeding the pH value of 9.5, preferably 9.0 and very particularly preferably 8.5, is prevented by adding a buffer substance before the start of the reduction.

8. Process according to Claims 1 to 7, characterised in that, when the reduction has ended, the sulphur present in the solution is removed by filtration and the 2-amino-4-nitrophenol is separated out in the filtrate by adjusting the pH value to 2 to 6, preferably 4.5 to 5.5.

9. Process according to Claims 1 to 7, characterised in that, when the reduction has ended, the sulphur present in the solution is filtered off and, after the filtration, impurities still present are dissolved by chemical reaction, preferably with an alkali metal sulphite, sulphur dioxide, alkali metal pyrosulphite, alkali metal bisulphite or alkali metal dithionite, and the 2-amino-4-nitrophenol is separated out by adjusting the pH value to 2 to 6, preferably 4.5 to 5.5.

10. Process according to Claims 1 to 9, characterised in that sodium hydrosulphide is used as the reducing agent.

## Revendications

1. Procédé pour préparer de l'amino-2 nitro-4 phénol par réduction du dinitro-2,4 phénol par l'hydrogène sulfuré en solution aqueuse alcaline à des températures de 20 à 100 °C, caractérisé en ce que pendant la réduction, on ne dépasse pas un pH de 9,5.

2. Procédé selon la revendication 1, caractérisé en ce que pendant la réduction, on ne dépasse pas un pH de 9,0, de préférence de 8,5.

3. Procédé selon l'une quelconque des revendications 1 ou 2, caractérisé en ce que pendant la réduction, on maintient un pH alcalin de 7 à 9,5, de préférence de 7,1 à 9,0, et tout particulièrement de 8,0 à 8,5.

4. Procédé selon l'une quelconque des revendications 1 à 3, caractérisé en ce qu'on effectue la réduction à une température de 50 à 80 °C.

5. Procédé selon l'une quelconque des revendications 1 à 4, caractérisé en ce qu'on utilise, pour la réduction d'1 mole de dinitro-2,4 phénol, 3 à 4 moles d'un sulfhydrate alcalin ou alcalino-terreux ou de sulfhydrate d'ammonium.

6. Procédé selon l'une quelconque des revendications 1 à 5, caractérisé en ce qu'on empêche le dépassement d'un pH de 9,5, de préférence de 9,0, tout particulièrement de 8,5, en ajoutant par portions successives ou en continu des substances fournissant des protons.

7. Procédé selon l'une quelconque des revendications 1 à 5, caractérisé en ce qu'on empêche le dépassement d'un pH de 9,5, de préférence de 9,0, tout particulièrement de 8,5, par addition d'une substance ayant un effet tampon avant le début de la réduction.

8. Procédé selon l'une quelconque des revendications 1 à 7, caractérisé en ce que lorsque la réduction est terminée, on élimine par filtration le soufre présent dans la solution et on amène dans le filtrat l'amino-2 nitro-4 phénol à se séparer par ajustement à un pH de 2 à 6, de préférence de 4 à 5,5.

9. Procédé selon l'une quelconque des revendications 1 à 7, caractérisé en ce que lorsque la réduction est terminée, on sépare par filtration le soufre présent dans la solution, et on amène en solution les impuretés encore présentes après la filtration à se séparer par une réaction chimique, de préférence avec un sulfite alcalin, de l'anhydride sulfureux, un pyrosulfite alcalin, un bisulfite alcalin ou un dithionite alcalin et on amène à se séparer l'amino-2 nitro-4 phénol en ajustant à un pH de 2 à 6, de préférence de 4,5 à 5,5.

10. Procédé selon l'une quelconque des revendications 1 à 9, caractérisé en ce qu'on utilise comme réducteur du sulfhydrate de sodium.